# EUROPÄISCHE PATENTSCHRIFT

(11) **EP 1 726 254 B1**
(45) Veröffentlichungstag und Bekanntmachung des Hinweises auf die Patenterteilung: **25.11.2009**
(21) Anmeldenummer: 06009823.3
(22) Anmeldetag: 12.05.2006
(51) Int. Cl.: A61B 1/00

(54) **Medizinisches Instrument für endoskopische Eingriffe**
Medical instrument for endoscopic operations
Dispositif medical pour interventions endoscopiques

(30) Priorität: 27.05.2005 DE 102005024352
(43) Veröffentlichungstag der Anmeldung: 29.11.2006
(73) Patentinhaber: Karl Storz GmbH & Co. KG, 78532 Tuttlingen (DE)
(72) Erfinder: Kupferschmid, Markus, 78756 Emmingen-Liptingen (DE); Walter, Christian, 78756 Emmingen-Liptingen (DE)
(74) Vertreter: Hofmeister, Frank

(56) Entgegenhaltungen:
- EP-A- 1 325 704
- DE-A- 19 933 015
- US-A- 4 736 733
- US-A- 4 986 622
- US-A- 5 518 501
- US-A- 5 621 830
- US-A1- 2002 125 857
- US-A1- 2005 080 342
- US-B1- 6 468 520

## Beschreibung

Die Erfindung betrifft ein medizinisches Instrument für endoskopische Eingriffe mit einem hohlen Schaft zur Aufnahme mindestens eines medizinischen Instruments und mit einer den Schaft tragenden Handhabe, wobei in der Handhabe mindestens ein sich im hohlen Schaft fortsetzender Kanal ausgebildet ist und die Handhabe über einen Köpplungsmechanismus auswechselbar am Schaft festlegbar ist, wobei der mindestens eine Kanal als Spül- und/oder Saugkanal ausgebildet ist, wobei im Bereich der Kopplungsstelle zwischen einem jeden handhabenseitigen Kanal und dem hohlen Schaft am Schaft und an der Handhabe ein Vorsprung und eine korrespondierende Ausnehmung zur Aufnahme des Vorsprungs ausgebildet sind sowie zwischen einem jeden handhabenseitigen Kanal und dem hohlen Schaft mindestens ein Dichtungselement angeordnet ist, über das jeder Vorsprung und die jeweilige korrespondierende Ausnehmung druckdicht miteinander verbunden sind.

Endoskopische Instrumente mit mindestens einem in der Handhabe ausgebildeten Kanal sind in verschiedenen Ausführungsformen bekannt. Bei diesen bekannten medizinischen Instrumenten sind der Schaft und die Handhabe nicht abnehmbar miteinander verbunden, so dass für einen Griff nur ein Schaft verwendbar ist. Neben dieser eingeschränkten Verwendbarkeit aufgrund der festen Verbindung von Schaft und Handhabe weisen die bekannten Instrumente den Nachteil auf, dass die durchgehenden Kanäle schlecht zu reinigen sind.

Ein gattungsgemäßes medizinisches Instrument ist beispielsweise aus der EP 1 325 704 A2 bekannt. Bei diesem bekannten medizinischen System sind der Instrumentenschaft und das Optikgehäuse über einen Schnellspannverschluss miteinander verbindbar. Der Schnellspannverschluss besteht dabei aus einer am proximalen Ende des Instrumentenschaftes angeordneten Aufnahmehülse, über die der festzulegende Instrumentenschaft auf das distale Ende des Optikgehäuses aufschiebbar ist. In der Aufnahmehülse sind von außen betätigbare federbelastete Rastelemente angeordnet, die nach vollständigem Aufschieben der Aufnahmehülse des Instrumentenschaftes auf das Optikgehäuse mit dem Optikgehäuse verrasten.

Dieser bekannte Schnellspannverschluss ermöglicht zwar ein schnelles und einfach zu handhabendes Verbinden und Lösen des Instrumentenschaftes mit dem Optikgehäuse, jedoch ist dieser bekannte Kopplungsmechanismus mit der Aufnahmehülse und den in der Aufnahmehülse angeordneten federbelasteten Rastelementen nur umständlich und mit großem Aufwand zu reinigen.

Aus der US 6 468 520 B1 ist weiterhin eine Vorrichtung zur lokalen Applikation von Polymermaterial bekannt. Diese bekannte Vorrichtung weist einen mit einem Durchgangskanal versehenen Handgriff auf, an dessen distalem Ende ein hohler Schaft über eine Schraubverbindung lösbar festgelegt ist.. Proximalseitig weist der Handgriff eine abnehmbare Endkappe auf, die über eine Steckverbindung am Handgriff festlegbar ist.

Des Weiteren ist aus der US 5 518 501 A der eine Vorrichtung zum Schutz eines Endoskops vor Kontamination bekannt. Diese Vorrichtung besteht aus einer mit einem Endoskop koppelbaren und einen Teil der Handhabe bildenden Kassettenreinheit, in der drei Kanäle ausgebildet sind, die sich durch die Kassette verstrecken und sich im mit dem Endoskop gekoppelten Zustand in im Schaft des Endoskops ausgebildeten Kanälen fortsetzen. Das Koppeln der Kassetteneinheit mit der eigentlichen Handhabe erfolgt über insgesamt vier Schraubverbindungen.

Davon ausgehend liegt der Erfindung die **Aufgabe** zugrunde, ein medizinisches Instrument für endoskopische Eingriffe so auszubilden, dass der Kopplungsmechanismus zum Verbinden von Handhabe und Schaft bei einfachem Aufbau eine gute und ausreichend feste Kopplung der miteinander zu verbindenden Bauteile gewährleistet.

Die **Lösung** dieser Aufgabenstellung ist erfindungsgemäß dadurch gekennzeichnet, dass der Kopplungsmechanismus als Rastverbindung mit zwei außen am Schaft gelagerten federbelasteten Kipphebeln ausgebildet ist und an der Handhabe und am Schaft Zentrierhilfen zum positionsgenauen Zusammenfügen der Handhabe und des Schaftes ausgebildet sind, wobei die Zentrierhilfen (22) als Stifte ausgebildet sind, die in entsprechende Aufnahmen an jeweils anderen Bauteil (2 oder 3) einfügbar sind und wobei die länge der Stifte so bemessen ist, dass sie den Schaft (2) und die Handhabe (3) schon relativ zu einander fixieren, bevor Schaft (2) und Handhabe (3) über den Kopplungsmechanismus mit einander gekoppelt werden.

Rastverbindungen zeichnen sich dadurch aus, dass sie bei einfachem Aufbau eine gute ausreichend feste Kopplung der miteinander zu verbindenden Bauteile gewährleisten und darüber hinaus einfach und schnell zu betätigen sind. Aufgrund der erfindungsgemäßen Trennbarkeit von Schaft und Handhabe ist es möglich, an einer Handhabe verschiedene Schäfte festlegen zu können. Ebenso können Schäfte beispielsweise mit individuell angepassten Handhaben versehen werden. Die Zerlegbarkeit der medizinischen Instrumente erleichtert darüber hinaus die Reinigbarkeit der solchermaßen ausgebildeten Instrumente. Das positionsgenau Zusammenfügen der Handhabe einerseits und des Schaftes andererseits wird durch die an der Handhabe und am Schaft ausgebildeten erfindungsgemäßen Zentrierhilfen erleichtert.

Die Vorsprünge und korrespondierenden Ausnehmungen im jeweils anderen Bauteil bewirken neben der Weiterführung des handhabenseitigen Kanals in den hohlen Schaft eine Zentrierung von Schaft und Handhabe zueinander, die das Koppeln der beiden Bauteile erleichtert.

Um zu gewährleisten, dass der Schaft und die Handhabe im Bereich der Kopplungsstelle des Spül-und/oder Saugkanals mit dem hohlen Schaft druckdicht miteinander verbunden sind, sind jeder Vorsprung und die jeweilige korrespondierende Ausnehmung über mindestens ein Dichtungselement druckdicht miteinander verbunden sind.

Zusätzlich zur Verbesserung der Druckdichtigkeit bewirken die vorzugsweise als O-Ringe ausgebildeten Dichtungselemente eine Verminderung der Strömungswiderstände in den Bereichen der Fügestellen zwischen Handhabe und Schaft, da die dort auftretenden Absätze oder Stufen durch die O-Ringe geglättet werden.

Um ein unbeabsichtigtes Trennen von Schaft und Handhabe zu verhindern, wird erfindungsgemäß vorgeschlagen, dass die Rastverbindung nur über das Betätigen eines gesonderten Entriegelungsmechanismus wieder lösbar ist.

Schließlich wird mit der Erfindung vorgeschlagen, dass der Entriegelungsmechanismus als mit der Rastverbindung in Wirkverbindung stehender Kipphebelmechanismus ausgebildet ist.

Gemäß einer ersten praktischen Ausführungsform, bei der der mindestens eine Kanal als mit Lichtleiterfasern bestückter Lichtkanal ausgebildet ist, wird erfindungsgemäß vorgeschlagen, dass zur Aufnahme der Lichtleitfasern im hohlen Schaft ein separater Kanal angeordnet ist, und dass die Lichtleiterfasern des handhabenseitigen Lichtkanals und des schaftseitigen Lichtkanals an den einander zugewandten Endflächen geschliffen und poliert sind. Ein solcher Lichtkanal ist notwendig, zur Beobachtung und Untersuchung über die Optik eines endoskopischen Instruments Licht in das Untersuchungsgebiet zu leiten. Das Schleifen und Polieren der miteinander zu koppelnden Lichtleiterendflächen ermöglicht eine weitestgehend ungehinderte Überleitung des Lichts vom handhabenseitigen Lichtkanal in den schaftseitigen Lichtkanal.

Die Lichtausbeute störende Reflexionen können erfindungsgemäß dadurch vermieden werden, dass im gekoppelten Zustand von Schaft und Handhabe die einander zugewandten Endflächen der Lichtleiterfasern des handhabenseitigen Lichtkanals und des schaftseitigen Lichtkanals exakt zentrisch zueinander ausgerichtet sind.

Um die Lichtverluste zu minimieren, die durch den verbleibenden kleinen Luftspalt zwischen den einander zugewandten Endflächen der Lichtleiterfasern des handhabenseitigen Lichtkanals und des schaftseitigen Lichtkanals verursacht werden, wird mit der Erfindung vorgeschlagen, dass im Bereich der Kopplungsstelle zwischen dem handhabenseitigen Lichtkanal und dem schaftseitigen Lichtkanal eine optisch wirksame Substanz, insbesondere ein optisches Gel, auf die Enden der Lichtleiterfasern auftragbar ist.

Zentrierfehler bei der zentrischen Ausrichtung der handhabenseitigen und schaftseitigen Lichtleiterfasern können weiterhin dadurch ausgeglichen werden, dass das Lichtleiterbündel in einem Lichtkanal einen größeren Durchmesser aufweist als das Lichtleiterbündel im mit diesem Lichtkanal zu koppelnden anderen Lichtkanal. Vorzugsweise weist dabei das Lichtleiterfaserbündel im handhabenseitigen Lichtkanal den größeren Durchmesser auf, da einerseits in der Handhabe mehr Platz zur Verfügung steht als im Instrumentenschaft und andererseits der lichtquellenseitige Lichtleiter vorteilhafterweise im Durchmesser größer ausgebildet ist, um eine volle Ausleuchtung des objektseitigen Lichtleiters zu gewährleisten.

Gemäß einer praktischen Ausführungsform der Erfindung sind der Spül- und Saugkanal als zwei getrennte Kanäle in der Handhabe ausgebildet. Vorteilhafterweise weist die Handhabe drei separate Kanäle auf, nämlich ein Lichtkanal, ein Spülkanal sowie ein Saugkanal, wobei der handhabenseitige Lichtkanal im Schaft in einem separaten Lichtkanal weitergeführt wird und die handhabenseitigen Spül- und Saugkanäle in den hohlen Schaft übergehen.

Weitere Merkmale und Vorteile der Erfindung ergeben sich anhand der zugehörigen Zeichnung, in der ein Ausführungsbeispiel eines erfindungsgemäßen medizinischen Instruments für endoskopische Eingriffe nur beispielhaft dargestellt ist. In der Zeichnung zeigt:
- Fig. 1: eine schematische Seitenansicht eines erfindungsgemäßen medizinischen Instruments für endoskopische Eingriffe im Längsschnitt;
- Fig. 2: eine vergrößerte ausschnittweise Darstellung des Details II gemäß Fig. 1 und
- Fig. 3: einen Schnitt entlang der Linie III-III gemäß Fig. 2.

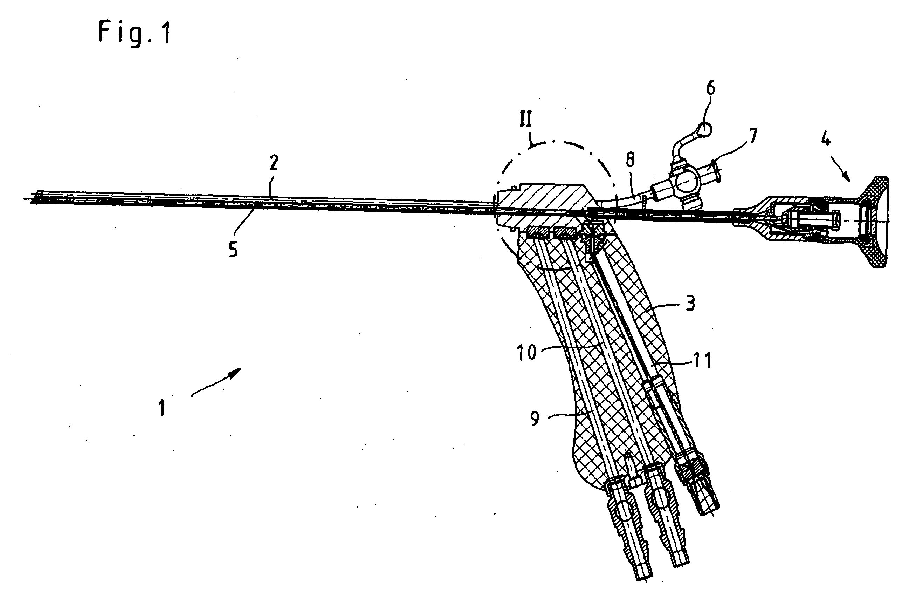

Die Abbildung Fig. 1 zeigt ein Endoskop 1, das im wesentlichen aus einem hohlen Schaft 2 und einer den Schaft 2 tragenden Handhabe 3 besteht. Zur Untersuchung des Operationsraums sowie zum Führen der Instrumente während einer Operation weist das Endoskop 1 ein aus verschiedenen Linsen aufgebautes optisches System auf, das proximal in einer Okulareinheit 4 endet. Zur Aufnahme der der Bildübertragung dienenden Linsensysteme ist im hohlen Schaft 2 ein optischer Kanal 5 angeordnet, der proximal in der Okulareinheit 4 mündet.

Proximalseitig weist das Endoskop 1 in der dargestellten geschnittenen Seitenansicht darüber hinaus einen über ein Ventil 6 verschließbaren Zugang 7 zu einem Arbeitskanal 8 auf, über den medizinische Instrumente, wie beispielsweise Schneid- und/oder Greifinstrumente, in den hohlen Schaft 2 und schließlich in das Operationsgebiet einführbar sind. Das Ventil 6 dient dazu, bei nicht eingesetztem Medizinischen Instrument den Arbeitskanal 8 druckdicht zu verschließen, um beispielsweise bei der Laparoskopie ein Entweichen des zur Ausbildung des Pneumoperitoneums dienenden Gases zu verhindern.

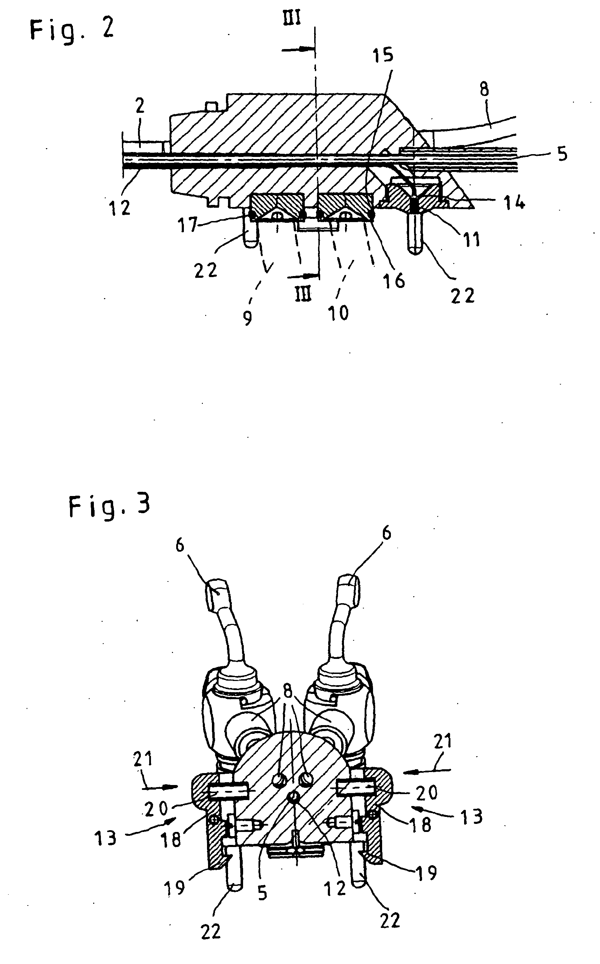

Selbstverständlich ist es möglich, dass das Endoskop 1 mehr als nur einen Arbeitskanal 8 zur Aufnahme von medizinischen Instrumenten aufweist, wie dies beispielsweise der Schnittdarstellung gemäß Fig. 3 zu entnehmen ist.

Wie weiterhin aus Fig. 1 und 2 ersichtlich, sind in der Handhabe 3 verschiedene Kanäle, nämlich ein Saugkanal 9, ein Spülkanal 10 sowie ein Lichtkanal 11 ausgebildet, die in den hohlen Schaft 2 übergehen. Während der Saugkanal 9 und der Spülkanal 10 frei in den hohlen Schaft 2 übergehen, setzt sich der handhabenseitige Lichtkanal 11 in einem Lichtkanal 12 im Schaft 2 fort.

Die mit den Kanälen 9, 10 und 11 versehene Handhabe 3 ist über einen in Fig. 3 dargestellten Kopplungsmechanismus 13 auswechselbar am Schaft 2 festlegbar.

Der Aufbau der Lichtkanäle 11 und 12 sowie des Kopplungsbereichs zwischen der Handhabe 3 und dem Schaft 2 ist insbesondere der vergrößerten Detailansicht gemäß Fig. 2 zu entnehmen.

Sowohl der handhabenseitige Lichtkanal 11 als auch der schaftseitige Lichtkanal 12 sind bestückt mit Lichtleiterfasern, über die Licht ausgehend von einer externen Lichtquelle hin zum Operationsgebiet leitbar ist, um über die Optik des Endoskops 1 das Operationsgebiet betrachten zu können. Um eine verlustarme Übertragung des Lichts von den Lichtleiterfasern des handhabenseitigen Lichtkanals 11 hin zu den Lichtleiterfasern des schaftseitigen Lichtkanals 12 zu gewährleisten, sind die einander zugewandten Endflächen der Lichtleiterfasern des handhabenseitigen Lichtkanals 11 und des schaftseitigen Lichtkanals 12 geschliffen und poliert und darüber hinaus exakt zentrisch zueinander ausgerichtet. Die zentrische Ausrichtung der Lichtfaserbündel zueinander verhindert das Auftreten störender Reflexionen.

Zur Überbrückung eines zwischen den einander zugewandten Endflächen der Lichtleiterfasern des handhabenseitigen Lichtkanals 11 und des schaftseitigen Lichtkanals 12 verbleibenden Luftspalts 14 und somit zur Minimierung aufgrund des Luftspalts 14 auftretender Lichtverluste ist eine optisch wirksame Substanz, wie beispielsweise ein optisches Gel, auf die Endflächen der Lichtleiterfasern auftragbar.

Wie aus Fig. 2 ersichtlich, weist das Lichtleiterfaserbündel im handhabenseitigen Lichtkanal 11 einen größeren Durchmesser auf als das Lichtleiterfaserbündel im schaftseitigen Lichtkanal 12. Durch die Ausbildung der Lichtleiterbündel mit unterschiedlichem Durchmesser ist es leicht möglich, etwaige Zentrierfehler beim Koppeln der Lichtkanäle 11 und 12 auszugleichen. Vorzugsweise weist dabei das Lichtleiterfaserbündel im handhabenseitigen Lichtkanal 11 den größeren Durchmesser auf, da einerseits in der Handhabe 3 mehr Platz zur Verfügung steht als im Instrumentenschaft 2 und andererseits der lichtquellenseitige Lichtleiter 11 vorteilhafterweise im Durchmesser größer ausgebildet ist, um eine volle Ausleuchtung des objektseitigen Lichtleiters 12 zu gewährleisten. Jedoch ist es selbstverständlich auch möglich, das Lichtleiterfaserbündel des schaftseitgen Lichtkanals 12 mit dem größerem Durchmesser zu versehen.

Das Koppeln von Handhabe 3 und Schaft 2 im Bereich des Saugkanals 9 und des Spülkanals 10 erfolgt über am Schaft 2 ausgebildete, ringförmige Vorsprünge 15, die im gekoppelten Zustand in korrespondierenden kreisförmigen Ausnehmungen 16 der Handhabe Aufnahme finden, wobei die kreisförmigen Ausnehmungen 16 die Kanäle 9 und 10 koaxial umgeben. Neben der Ausbildung des Übergangs der Kanäle 9 und 10 zum hohlen Schaft 2 dienen die Vorsprünge 15 und Ausnehmungen 16 zum gegenseitigen Zentrieren von Schaft 2 und Handhabe 3 während des Koppelns.

Um zu gewährleisten, dass die Koppelstelle von Schaft 2 und Handhabe 3 im Bereich der Kanäle 9 und 10 druckdicht ausgebildet ist, sind die ringförmigen Vorsprünge 15 mit als O-Ringen 17 ausgebildeten Dichtungselementen bestückt. Alternativ zu der dargestellten konstruktiven Ausgestaltung der Vorsprünge 15 und Ausnehmungen 16 ist es selbstverständlich auch möglich, die Vorsprünge an der Handhabe 3 und die Ausnehmungen 16 zur Aufnahme der Vorsprünge 15 am Schaft 2 auszubilden. Ebenso besteht die Möglichkeit, die Dichtungselemente, beispielsweise die O-Ringe 17, in den Ausnehmungen 16 anzuordnen.

Zusätzlich zur Verbesserung der Druckdichtigkeit bewirken die O-Ringe 17 eine Verminderung der Strömungswiderstände in den Bereichen der Fügestellen zwischen Handhabe 3 und Schaft 2, da die dort auftretenden Absätze oder Stufen durch die O-Ringe 17 geglättet werden.

Das gegenseitige Fixieren von Schaft 2 und Handhabe 3 erfolgt bei der dargestellten Ausgestaltungsform über den in Fig. 3 dargestellten, als Rastverbindung ausgebildeten Kopplungsmechanismus 13. Wie aus Fig. 3 ersichtlich, besteht die dargestellte Rastverbindung aus zwei am Schaft 2 gelagerten Kipphebeln 18, an deren der Handhabe 3 zugewandten freien Enden Rastnasen 19 ausgebildet sind, die im gekoppelten Zustand in entsprechende Rastaufnahmen in der Handhabe 3 eingreifen.

Um ein unbeabsichtigtes Lösen der Rastverbindung zu verhindern, sind die Kipphebel 18 so ausgebildet, dass die Verrastung nur durch aktives Betätigen beider Kipphebel 18 wieder aufhebbar ist. Hiezu sind die Kipphebel 18, wie aus der Abbildung Fig. 3 ersichtlich, so ausgebildet, dass pro Kipphebel 18 ein Federelement 20 vorgesehen ist, das mit dem jeweiligen Kipphebel 18 in Wirkverbindung steht. Sobald auf die freien Enden der Kipphebel 18 in Richtung der Pfeile 21 wirkende Druckkräfte ausgeübt werden, werden die Rastnasen 19 aus den Rastaufnahmen an der Handhabe 3 herausgehoben, so dass der Schaft 2 von der Handhabe 3 abgenommen werden kann.

Das positionsgenau Zusammenfügen der Handhabe 3 einerseits und des Schaftes 2 andererseits wird bei der dargestellten Ausführungsform dadurch erleichtert, dass am Schaft 2 als Stifte ausgebildete Zentrierhilfen 22 angeordnet sind, die in korrespondierende Aufnahmen in der Handhabe 3 eingefügt werden. Die Länge der Stifte, die auch nur an der Handhabe 3 oder an beiden Bauteilen 2 und 3 angeordnet sein können, ist so bemessen, dass die in die entsprechenden Aufnahmen im jeweils anderen Bauteil eingesetzten Stifte die Handhabe 3 und den Schaft 2 schon relativ zueinander fixieren, bevor die Bauteile 2 und 3 über den Kopplungsmechanismus 13 miteinander gekoppelt werden.

Ein wie beschrieben ausgebildetes medizinisches Instrument für endoskopische Eingriffe zeichnet sich dadurch aus, dass der Schaft 2 und die Handhabe 3 lösbar miteinander verbunden sind.

Neben der besseren Reinigbarkeit der beiden separierten Bauteile 2 und 3 zeichnet sich ein solchermaßen ausgebildetes medizinisches Instrument durch eine größere Flexibilität aus. So ist es beispielsweise möglich eine Handhabe 3 für verschiedene Schäfte 2 zu verwenden, oder aber einen Schaft 2 mit unterschiedlichen Handhaben 3 zu versehen, die beispielsweise an besondere individuelle Anforderungen/Wünsche des Operateurs angepasst sind. Ohne ein vollständiges Instrument bereitstellen zu müssen, ist es so möglich, ein ausgesprochen vielseitig einsetzbares medizinisches Instrument zur Verfügung zu stellen.

### Bezugszeichenliste

- 1: Endoskop
- 2: Schaft
- 3: Handhabe
- 4: Okulareinheit
- 5: optischer Kanal
- 6: Ventil
- 7: Zugang
- 8: Arbeitskanal
- 9: Saugkanal
- 10: Spülkanal
- 11: Lichtkanal (Handhabe)
- 12: Lichtkanal (Schaft)
- 13: Kopplungsmechanismus
- 14: Luftspalt
- 15: Vorsprung
- 16: Ausnehmung
- 17: O-Ring
- 18: Kipphebel
- 19: Rastnase
- 20: Federelement
- 21: Pfeil (Druckkraft)
- 22: Zentrierhilfe

## Patentansprüche

1. Medizinisches Instrument für endoskopische Eingriffe mit einem hohlen Schaft (2) zur Aufnahme mindestens eines medizinischen Instruments und mit einer den Schaft (2) tragenden Handhabe (3), wobei in der Handhabe (3) mindestens ein sich im hohlen Schaft (2) fortsetzender Kanal (9, 10, 11) ausgebildet ist und die Handhabe (3) über einen Kopplungsmechanismus (13) auswechselbar am Schaft (2) festlegbar ist, wobei der mindestens eine Kanal als Spül- und/oder Saugkanal (10, 9) ausgebildet ist, wobei im Bereich der Kopplungsstelle zwischen einem jeden handhabenseitigen Kanal (9, 10) und dem hohlen Schaft (2) am Schaft (2) und an der Handhabe (3), ein Vorsprung (15) und eine korrespondierende Ausnehmung (16) zur Aufnahme des Vorsprungs (15) ausgebildet sind sowie zwischen einem jeden handhabenseitigen Kanal (9, 10) und dem hohlen Schaft (2) mindestens ein Dichtungselement angeordnet ist, über das jeder Vorsprung (15) und die jeweilige korrespondierende Ausnehmung (16) druckdicht miteinander verbunden sind
**dadurch gekennzeichnet,**
**dass** der Kopplungsmechanismus (13) als Rastverbindung mit zwei außen am Schaft (2) gelagerten federbelasteten Kipphebels (18) ausgebildet ist und an der Handhabe (3) und/oder am Schaft (2) Zentrierhilfen (22) zum positionsgenauen Zusammenfügen der Handhabe (3) und des Schaftes (2) ausgebildet sind wobei die Zentrierhilfen (22) als Stifte ausgebildet sind, die in entsprechende Aufnahmen an jeweils anderen Bauteil (2 oder 3) einfügbar sind und wobei die länge der Stifte so bemessen ist, dass sie den Schaft (2) und die Handhabe (3) schon relativ zu einander fixieren, bevor Schaft (2) und Handhabe (3) über den Kopplungsmechanismus mit einander gekoppelt werden.

2. Medizinisches Instrument nach Anspruch 1, **dadurch gekennzeichnet, dass** die Kipphebel (18) an ihren der Handhabe (3) zugewandten freien Enden Rastnasen (19) aufweisen.

3. Medizinisches Instrument nach Anspruch 2, **dadurch gekennzeichnet, dass** sich die Kipphebel (18) mit ihren der Handhabe (3) abgewandten freien Enden über jeweils ein Federelement (20) am Schaft (2) abstützen.

4. Medizinisches Instrument nach einem der Ansprüche 1 bis 3, **dadurch gekennzeichnet, dass** das mindestens eine Dichtungselement als O-Ring (17) ausgebildet ist.

5. Medizinisches Instrument nach einem der Ansprüche 1 bis 4, **dadurch gekennzeichnet, dass** die Rastverbindung über einen Entriegelungsmechanismus lösbar ist.

6. Medizinisches Instrument nach Anspruch 5, **dadurch gekennzeichnet, dass** der Entriegelungsmechanismus als mit der Rastverbindung in Wirkverbindung stehender Kipphebelmechanismus ausgebildet ist.

7. Medizinisches Instrument nach einem der Ansprüche 1 bis 6, wobei der mindestens eine Kanal als mit Lichtleiterfasern bestückter Lichtkanal (11) ausgebildet ist, **dadurch gekennzeichnet, dass** zur Aufnahme der Lichtleitfasern im hohlen Schaft (2) ein separater Kanal (12) angeordnet ist, und dass die Lichtleiterfasern des handhabenseitigen Lichtkanals (11) und des schaftseitigen Lichtkanals (12) an den einander zugewandten Endflächen geschliffen und poliert sind.

8. Medizinisches Instrument nach Anspruch 7, **dadurch gekennzeichnet, dass** im gekoppelten Zustand die einander zugewandten Endflächen der Lichtleiterfasern des handhabenseitigen Lichtkanals (11) und des schaftseitigen Lichtkanals (12) zentrisch zueinander ausgerichtet sind.

9. Medizinisches Instrument nach Anspruch 7 oder 8 **dadurch gekennzeichnet, dass** im Bereich der Kopplungsstelle zwischen dem handhabenseitigen Lichtkanal (11) und dem schaftseitigen Lichtkanal (12) eine optisch wirksame Substanz, insbesondere ein optisches Gel, auf die Enden der Lichtleiterfasern auftragbar ist.

10. Medizinisches Instrument nach einem der Ansprüche 7 bis 9, **dadurch gekennzeichnet, dass** das Lichtleiterbündel in einem Lichtkanal (11 oder 12) einen größeren Durchmesser aufweist als das Lichtleiterbündel im mit diesem Lichtkanal (11 oder 12) zu koppelnden anderen Lichtkanal (12 oder 11).

11. Medizinisches Instrument nach einem der Ansprüche 1 bis 10, **dadurch gekennzeichnet, dass** in der Handhabe (3) zwei getrennte, als Spül- und Saugkanal dienende Kanäle (10, 9) ausgebildet sind.

12. Medizinisches Instrument nach einem der Ansprüche 1 bis 10, **dadurch gekennzeichnet, dass** in der Handhabe (3) drei Kanäle, nämlich ein Lichtkanal (11), ein Spülkanal (10) sowie ein Saugkanal (9) ausgebildet sind.

## Claims

1. Medical instrument for endoscopic interventions, with a hollow shaft (2) for receiving at least one medical instrument, and with a handle (3) that supports the shaft (2), which handle (3) contains at least one channel (9, 10, 11) that continues within the hollow shaft (2), and which handle (3) can be secured releasably on the shaft (2) via a coupling mechanism (13), the at least one channel is designed as a flushing and/or suction channel (10, 9), and, in the area of the coupling site between each handle-side channel (9, 10) and the hollow shaft (2), a projection (15) and a corresponding recess (16) for receiving the projection (15) are formed on the shaft (2) and on the handle (3), and, arranged between each handle-side channel (9, 10) and the hollow shaft (2), there is at least one sealing element via which each projection (15) and the respective corresponding recess (16) are connected to each other in a pressure-tight manner, **characterized in that** the coupling mechanism (13) is designed as a locking connection with two spring-loaded tilting levers (18) mounted on the shaft (2), and centring aids (22) for joining the handle (3) and shaft (2) together in an exact position are formed on the handle (3) and/or on the shaft (2), which centring aids (22) are designed as pins that can be inserted into corresponding recesses on the respective other component (2 or 3), and the length of the pins is such that they already fix the shaft (2) and the handle (3) relative to each other before shaft (2) and handle (3) are coupled to each other via the coupling mechanism.

2. Medical instrument according to Claim 1, **characterized in that** the tilting levers (18) have locking lugs (19) at their free ends directed towards the handle (3).

3. Medical instrument according to Claim 2, **characterized in that** the free ends of the tilting levers (18) directed away from the handle (3) are supported on the shaft (2) in each case via a spring element (20).

4. Medical instrument according to one of Claims 1 to 3, **characterized in that** the at least one sealing element is designed as an 0-ring (17).

5. Medical instrument according to one of Claims 1 to 4, **characterized in that** the locking connection is releasable via an unlocking mechanism.

6. Medical instrument according to Claim 5, **characterized in that** the unlocking mechanism is designed as a tilting lever mechanism operatively connected to the locking connection.

7. Medical instrument according to one of Claims 1 to 6, in which the at least one channel is designed as a light channel (11) provided with light-conducting fibres, **characterized in that** a separate channel (12) is arranged in the hollow shaft (2) in order to receive the light-conducting fibres, and **in that** the light-conducting fibres of the handle-side light channel (11) and of the shaft-side light channel (12) are ground and polished on the mutually facing end surfaces.

8. Medical instrument according to Claim 7, **characterized in that**, in the coupled state, the mutually facing end surfaces of the light-conducting fibres of the handle-side light channel (11) and of the shaft-side light channel (12) are aligned centrally to one another.

9. Medical instrument according to Claim 7 or 8, **characterized in that**, in the area of the coupling site between the handle-side light channel (11) and the shaft-side light channel (12), an optically active substance, in particular an optical gel, can be applied on the ends of the light-conducting fibres.

10. Medical instrument according to one of Claims 7 to 9, **characterized in that** the fibre optic bundle in one light channel (11 or 12) has a greater diameter than the fibre optic bundle in the other light channel (12 or 11) that is to be coupled to this light channel (11 or 12).

11. Medical instrument according to one of Claims 1 to 10, **characterized in that** two separate channels (10, 9) are formed in the handle (3) and serve as flushing and suction channels.

12. Medical instrument according to one of Claims 1 to 10, **characterized in that** three channels are formed in the handle (3), namely a light channel (11), a flushing channel (10), and a suction channel (9).

## Revendications

1. Instrument médical pour des interventions endoscopiques, comprenant un corps allongé creux (2) destiné à recevoir au moins un instrument médical, et une poignée (3) portant le corps allongé (2),
instrument dans lequel dans la poignée (3) est réalisé au moins canal (9, 10, 11) se poursuivant dans le corps allongé creux (2), et la poignée (3) peut être fixée de manière interchangeable sur le corps allongé (2) par l'intermédiaire d'un mécanisme de couplage (13),
dans lequel ledit au moins un canal est réalisé en tant que canal de rinçage et/ou d'aspiration (10, 9),
dans lequel sont formés, dans la région de la zone de couplage entre chaque canal (9, 10) de la poignée et le corps allongé creux (2), sur le corps allongé (2) et sur la poignée (3), une protubérance (15) et un évidement... (16) correspondant destiné à recevoir la protubérance (15),
et dans lequel, entre chaque canal (9, 10) de la poignée et le corps allongé creux (2), est agencé au moins un élément d'étanchéité par l'intermédiaire duquel chaque protubérance (15) et l'évidement (16) respectivement correspondant sont reliés mutuellement de manière étanche à la pression,
**caractérisé en ce que** le mécanisme de couplage (13) est réalisé sous forme de liaison à encliquetage avec deux leviers basculants (18) chargés par ressort, qui sont montés à l'extérieur sur le corps allongé (2), et sur la poignée (3) et/ou sur le corps allongé (2) sont formés des éléments d'aide au centrage (22) pour la réunification en position précise de la poignée (3) et du corps allongé (2), les éléments d'aide au centrage (22) étant réalisés sous forme de goupilles qui peuvent être insérées dans des logements de réception correspondants sur l'autre pièce respective (2 ou 3), et la longueur des goupilles étant dimensionnée de manière à ce qu'elles fixent déjà relativement l'un par rapport à l'autre le corps allongé (2) et la poignée (3) avant que le corps allongé (2) et la poignée (3) soient couplées mutuellement par l'intermédiaire du mécanisme de couplage.

2. Instrument médical selon la revendication 1, **caractérisé en ce que** les leviers basculants (18) présentent, à leurs extrémités libres dirigées vers la poignée (3), des talons d'encliquetage (19).

3. Instrument médical selon la revendication 2, **caractérisé en ce que** les leviers basculants (18) s'appuient sur le corps allongé (2), avec leurs extrémités éloignées de la poignée (3), chacun par l'intermédiaire d'un élément de ressort (20).

4. Instrument médical selon l'une des revendications 1 à 3, **caractérisé en ce que** ledit au moins un élément d'étanchéité est réalisé sous forme de joint torique (17).

5. Instrument médical selon l'une des revendications 1 à 4, **caractérisé en ce que** la liaison à encliquetage peut être interrompue à l'aide d'un mécanisme de déverrouillage.

6. Instrument médical selon la revendication 5, **caractérisé en ce que** le mécanisme de déverrouillage est réalisé sous la forme d'un mécanisme à levier basculant en liaison interactive avec la liaison à encliquetage.

7. Instrument médical selon l'une des revendications 1 à 6, ledit au moins un canal étant réalisé en tant que canal de lumière (11) équipé de fibres optiques, **caractérisé en ce que** pour recevoir les fibres optiques, dans le corps allongé creux (2) est formé un canal séparé (12), et **en ce que** les fibres optiques du canal de lumière (11) de la poignée et du canal de lumière (12) du corps allongé, sont rectifiées et polies aux surfaces d'extrémité mutuellement adjacentes.

8. Instrument médical selon la revendication 7, **caractérisé en ce que** dans l'état couplé, les surfaces d'extrémité mutuellement adjacentes des fibres optiques du canal de lumière (11) de la poignée et du canal de lumière (12) du corps allongé sont orientées de manière à être centrées réciproquement.

9. Instrument médical selon la revendication 7 ou la revendication 8, **caractérisé en ce que** dans la région de la zone de couplage entre le canal de lumière (11) de la poignée et le canal de lumière (12) du corps allongé, une substance active sur le plan optique, notamment un gel optique, peut être appliqué sur les extrémités des fibres optiques.

10. Instrument médical selon l'une des revendications 7 à 9, **caractérisé en ce que** le faisceau de fibres optiques dans un canal de lumière (11 ou 12) présente un diamètre plus grand que le faisceau de fibres optiques dans l'autre canal de lumière (12 ou 11) devant être couplé au canal de lumière cité en premier (11 ou 12).

11. Instrument médical selon l'une des revendications 1 à 10, **caractérisé en ce que** dans la poignée (3) sont réalisés deux canaux (10, 9) séparés, en tant que canal de rinçage et d'aspiration.

12. Instrument médical selon l'une des revendications 1 à 10, **caractérisé en ce que** dans la poignée (3) sont réalisés trois canaux, à savoir un canal de lumière (11), un canal de rinçage (10), ainsi qu'un canal d'aspiration (9).
